(19) Europäisches Patentamt
European Patent Office
Office européen des brevets

(11) Veröffentlichungsnummer: **0 080 554**
**B1**

(12) **EUROPÄISCHE PATENTSCHRIFT**

(45) Veröffentlichungstag der Patentschrift: **07.01.87**

(51) Int. Cl.[4]: **C 07 D 235/32**, C 07 D 235/30

(21) Anmeldenummer: **82106803.8**

(22) Anmeldetag: **17.10.80**

(60) Veröffentlichungsnummer der früheren Anmeldung nach Art. 76 EPÜ: **0027646**

(54) **Verfahren zur Herstellung von 5(6)-Thiocyan-benzimidazol-Derivaten.**

(30) Priorität: **03.06.80 HU 138780**
**17.09.80 HU 138780**

(43) Veröffentlichungstag der Anmeldung: **08.06.83 Patentblatt 83/23**

(45) Bekanntmachung des Hinweises auf die Patenterteilung: **07.01.87 Patentblatt 87/2**

(84) Benannte Vertragsstaaten: **AT CH DE FR GB IT LI**

(56) Entgegenhaltungen:
**EP - A - 0 003 951**
**DE - A - 2 041 324**
**DE - A - 2 363 348**
**DE - A - 2 454 632**
**US - A - 4 002 640**

(73) Patentinhaber: **CHINOIN Gyogyszer és Vegyészeti Termékek Gyára RT., To utca 1-5, H-1045 Budapest IV (HU)**

(72) Erfinder: **Gönczi, Csaba, Dr., Dipl.Ing.Chem., Lisznyai u. 5, H-1016 Budapest (HU)**
Erfinder: **Korbonits, Dezsö, Dr., Dipl.Ing.Chem., Vérhalom u. 27/D, H-1025 Budapest (HU)**
Erfinder: **Pálosi, Endre, Dipl.Ing.Chem., Pasaréti ut 114/a, H-1026 Budapest (HU)**
Erfinder: **Kiss, Pál, Dr., Dipl.Ing.Chem., Vetés u. 82, H-1046 Budapest (HU)**
Erfinder: **Héja, Gergely, Dr., Dipl.Ing.Chem., Sollner u. 27, H-1131 Budapest (HU)**
Erfinder: **Kun, Judit, Dipl.Ing.Chem., Gyenes u. 17, H-1032 Budapest (HU)**
Erfinder: **Szomor geb. Wundele, Mária, Dipl.Ing.Chem., Fö u. 21, H-1011 Budapest (HU)**
Erfinder: **Szvoboda geb. Kanzel, Ida, Dipl. Ing. Chem., Fogarasi u. 85 1/5, H-1141 Budapest (HU)**
Erfinder: **Márványos, Ede, Dipl.Ing.Chem., Wesselényi u. 69, H-1077 Budapest (HU)**
Erfinder: **Kovács geb. Mindler, Vera Dipl.Ing.Chem., Rona park 4, H-1142 Budapest (HU)**
Erfinder: **Nagy geb. Korányi, Livia, Dipl.Ing.Chem., Frankel L.u. 24, H-1027 Budapest (HU)**

(74) Vertreter: **Lotterhos, Hans Walter, Dr.-Ing., Lichtensteinstrasse 3, D-6000 Frankfurt am Main 1 (DE)**

**EP 0 080 554 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 5(6)-Thiocyano-benzimidazol-Derivaten der allgemeinen Formel I

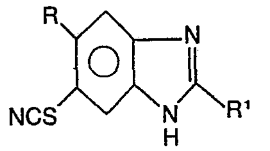

(I)

worin

R Wasserstoff, Halogen, $C_{1-4}$-Alkyl, $C_{1-3}$-Alkoxy oder Trifluormethyl,

$R^1$ Amino oder $C_{1-4}$-Alkoxycarbonylamino bedeuten, sowie deren Salzen.

Die 5(6)-Thiocyano-benzimidazol-Derivate der allgemeinen Formel I stellen Zwischenprodukte für die Herstellung der als Anthelmintica bekannten Benzimidazolderivate der allgemeinen Formel

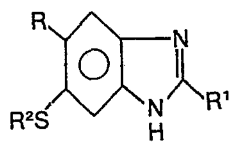

dar,

worin

R und $R^1$ die oben angegebene Bedeutung haben und

$R^2$ ein ionisch gebundenes Metallatom, ein ionisch oder kovalent gebundenes Wasserstoffatom, $C_{1-6}$-Alkyl, Alkenyl oder Alkinyl mit jeweils bis zu 6 C-Atomen, oder Cyclohexyl, Benzyl oder 2-Phenyläthyl bedeutet,

wobei ein 5(6)-Thiocyano-benzimidazol-Derivat der allgemeinen Formel I durch Behandeln mit einer nucleophilen, organischen oder anorganischen Base in eine Verbindung der allgemeinen Formel

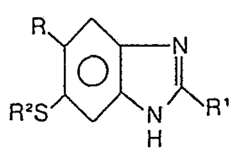

worin

R und $R^1$ die oben angegebene Bedeutung haben und

$R^2$ ein ionisch gebundenes Metallatom, ein ionisch oder kovalent gebundenes Wasserstoffatom bedeutet,

übergeführt wird, die gegebenenfalls mit einem Alkylierungsmittel der allgemeinen Formel

$$R^{2''} - Q$$

worin

$R^{2''}$ $C_{1-6}$-Alkyl, Alkenyl oder Alkinyl mit jeweils bis zu 6 C-Atomen, Cyclohexyl, Benzyl oder 2-Phenyläthyl und

Q Hydroxy, Halogen, ½ $SO_4$, ½ $PO_3$, ⅓ $PO_4$, $SO_3C_6H_5$ oder $SO_3C_6H_4CH_3$ bedeuten,

umgesetzt wird.

Aus der US-PS 4 080 461 ist es bereits bekannt, zur Herstellung von 5(6)-Thiocyano-benzimidazol-Derivaten der allgemeinen Formel I in der 1. Verfahrensstufe 1-Amino-2-nitro-4-thiocyano-benzol in konzentrierter Salzsäure bei −40 °C mit überschüssigem Zinn(II)-chlorid zum 1,2-Diamino-4-thiocyano-benzol zu reduzieren, das dann – nach Extraktion mit Chloroform und Isolierung in der 2. Verfahrensstufe durch Umsetzung mit 1,3-bis-(Alkoxycarbonyl)-S-methyl-isothioharnstoff in einem wässrigen, essigsauren Medium unter Mercaptanentwicklung in das 5(6)-Thiocyano-benzimidazol-Derivat übergeführt wird (vgl. US-PS 4 080 461, Beispiel 1).

Die Anwendung der ungewähnlich tiefen Reaktionstemperatur von −40 °C in der Reduktionsstufe 1 sowie die Wahl des Cyclisierungsmittels trotz der damit verbundenen Umweltbelastung durch die Mercaptanentwicklung in der Cyclisierungsstufe 2 war trotz der damit verbundenen verfahrenstechnischen Schwierigkeiten für notwendig gehalten worden, weil aus der Literatur bekannt war, dass die Thiocyangruppe sehr reaktionsfähig ist, zu verschiedenen Umwandlungen neigt und im basischen wie sauren Medium zur Bildung von Disulfiden und/oder Thiolverbindungen neben anderen Produkten führt. [Houben-Weil, Methoden der organischen Chemie, Bd. IX, S. 16/17, 866, Thieme Verl. Stuttgart (1955); Chem. Rev. 49, 77 (1951)].

Da es weiterhin bekannt war, dass die Thiocyangruppe auch auf Reduktions- und Hydrolysebedingungen sehr empfindlich reagiert [R. G. Guy, The Chemistry of Cyanates and their Thioderivates, Teil 2, S. 871 (1974)] war nicht zu erwarten, dass sich die Nitrogruppe des 1-Amino-2-nitro-4-thiocyano-benzols bei Anwendung üblicher Reduktionsbedingungen zur Aminogruppe reduzieren lassen würde, ohne dass auch die Thiocyangruppe unter Bildung unerwünschter Thiol- und/oder Disulfidnebenprodukte in Reaktion treten würde. Die katalytische Hydrierung in einem neutralen Medium schied allein schon deshalb aus, weil die Thiocyangruppe ein starkes Katalysatorgift ist.

Es wurde in überraschender Weise gefunden, dass sich die 5(6)-Thiocyano-benzimidazol-Derivate der allgemeinen Formel I in sehr einfacher und auch in industriellem Massstab durchführbaren Weise in guter Ausbeute herstellen lassen, wenn man eine Verbindung der allgemeinen Formel

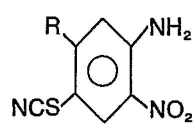

(II)

worin

R die oben angegebene Bedeutung hat,

mit Eisen zur Verbindung der allgemeinen Formel III

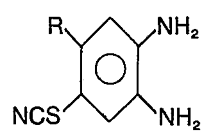

(III)

worin
R die oben angegebene Bedeutung hat,
selektiv reduziert, die entweder mit einem Cyanamid der allgemeinen Formel IV

$$R^3 - NHCN \quad (IV)$$

worin
$R^3$ Wasserstoff oder $C_{1-4}$-Alkoxycarbonyl bedeutet,
oder mit einem Halogencyan der allgemeinen Formel V

$$HlgCN \quad (V)$$

worin
Hlg Chlor oder Brom bedeutet,
umgesetzt wird.

Die Reduktion der Verbindungen der allgemeinen Formel II mit Eisen wird zweckmässig in einem wässrigen-organischen, heterogenen Lösungsmittelsystem bei neutralem oder schwach saurem pH durchgeführt.

Die Cyclisierung mit einem Cyanamid der allgemeinen Formel IV wird zweckmässig in einem wässrigen oder wässrig-organischen Lösungsmittelmedium in Gegenwart einer anorganischen Säure durchgeführt und die mit einem Halogencyan der allgemeinen Formel V in einem organischen Lösungsmittel.

Es stellte sich in überraschender Weise heraus, dass die Thiocyangruppe bei der Reduktion der Nitrogruppe gemäss der Erfindung weder eine reduktive noch eine hydrolytische Umwandlung erleidet, was den Einsatz schwer zugänglicher Reagentien und die Anwendung sehr niedriger Temperaturen überflüssig macht. Es wurde weiterhin gefunden, dass sich die Thiocyangruppe in verhältnismässig stark saurem Medium auch unter Erwärmen nicht zersetzt, so dass man bei der Ausbildung des Benzimidazolringes die Verwendung der schwer zugänglichen 1,3-bis-(Alkoxycarbonyl)-S-methyl-isothioharnstoffe umgehen kann.

Nach einer vorteilhaften Ausführungsform des Verfahrens wird die Reduktion – auf die Nitrogruppe bezogen – mit 2,25 bis 3,5 g-Atom Eisen durchgeführt, das vorzugsweise in feinteiliger Form verwendet wird. Zweckmässig wird so verfahren, dass man das Eisen einer aktivierenden Vorbehandlung mit 0,005 bis 0,2 Mol Säure oder Salz, vorzugsweise zwischen Raumtemperatur und 100 °C, insbesondere zwischen 90 und 95 °C, unterwirft. Als Säure werden Schwefelsäure und Salzsäure und als Salz Ammoniumchlorid, Eisen(II)-sulfat, Eisen(III)-chlorid, Ammoniumsulfat oder Calciumchlorid bevorzugt. Bei der Reduktion können als Reaktionsmedium – je nach Löslichkeit der Ausgangsstoffe – mit Wasser mischbare, organische Lösungsmittel, wie Aceton, Dioxan, niedrige Alkohole oder Wasser allein, vorzugsweise Methyl- oder Äthylalkohol, verwendet werden. Die Reduktion wird bei Temperaturen zwischen 20 und 100 °C, vorzugsweise beim Siedepunkt des verwendeten Lösungsmittels, vorgenommen. Sie kann je nach den Ausgangsverbindungen und Reaktionsbedingungen einige Minuten aber auch 1 bis 2 Stunden in Anspruch nehmen. Zweckmässig werden die Menge der Säure oder des Salzes und die Reaktionsbedingungen so gewählt, dass die Reduktionsdauer 0,25 bis 1 Stunde beträgt.

Die Cyclisierung der Thiocyano-o-phenylendiamine der allgemeinen Formel III mit Cyanamiden der allgemeinen Formel IV wird vorzugsweise in einem wässrig sauren Medium bei einem pH-Wert zwischen 2 und 6, vorzugsweise zwischen 3 und 4, vorgenommen. Während des Ringschlusses wird der Lösung vorzugsweise wässrige Säure zugegeben, um den pH-Wert der Lösung in dem gewünschten sauren Bereich zu halten. Die Reaktion wird zweckmässig bei Temperaturen zwischen 25 und 100 °C, vorzugsweise zwischen 70 und 100 °C, durchgeführt. Als Säure können organische oder anorganische Säuren, vorzugsweise Salzsäure oder Schwefelsäure, verwendet werden.

Die Reaktion wird zweckmässig in der Weise durchgeführt, dass man ein Thiocyano-o-phenylendiamin der allgemeinen Formel III in einer wässrigen Säure, vorzugsweise 5 n Salzsäure, unter Erwärmen löst und die so erhaltene Lösung mit einer wässrigen Lösung des Calciumsalzes einer Verbindung der allgemeinen Formel IV versetzt. Man kann aber auch so verfahren, dass man das Calciumsalz des Carbalkoxycyanamids der allgemeinen Formel IV nicht vorher isoliert, sondern für den Ringschluss das in dem wässrigen Medium durch Umsetzung von technischem Calciumcyanamid mit einem Chlorameisensäurealkylester in situ gebildete Calciumsalz des Carbalkoxycyanamids verwendet. Nach einer vorteilhaften Ausführungsform des Verfahrens wird das Thiocyano-o-phenylendiamin der allgemeinen Formel III in einer Lösung von Wasser mit einem organischen Lösungsmittel (vorzugsweise Äthanol) mit einem Cyanamid der allgemeinen Formel IV umgesetzt.

Die Umsetzung der Thiocyano-o-phenylendiamine der allgemeinen Formel III mit Halogencyanen wird zweckmässig in einem geeigneten organischen Lösungsmittel, vorteilhaft in einem niedrigen Alkohol, z.B. Methanol, Äthanol, Propanol oder Isopropanol, vorzugsweise Äthanol, bei einer Temperatur von 0 bis 40 °C vorgenommen. Das Reaktionsgemisch wird mit einem Alkalihydroxyd, vorteilhaft Natriumhydroxyd oder Kaliumhydroxyd, oder einem Alkalicarbonat, z.B. Natrium- oder Kaliumcarbonat, behandelt, wobei 5(6)-Thiocyano-benzimidazol-Derivate der allgemeinen Formel I erhalten werden.

Mit dem Verfahren der Erfindung werden die verfahrenstechnischen Nachteile des Verfahrens der US-PS 4 080 461 in sehr einfacher Weise umgangen. Die benötigten Ausgangsverbindungen sind auch in grossen Mengen leicht zugänglich; bei der Reduktion werden die Thiocyano-o-phenylendiamine in guten Ausbeuten (bis zu etwa 80%) erhalten und die Cyclisierung verläuft praktisch ohne Bildung von Nebenprodukten in Ausbeuten bis zu 90%. Da sich die Thiocyano-o-phenylendiamine leicht reinigen lassen, kann man bei der Cyclisierung von reinem Thiocyano-o-phenylendiamin die 5(6)-Thiocyano-benzimidazol-Derivate der allgemeinen Formel I bereits in so reinem Zustand erhalten, dass sie ohne weitere Reinigung zu den weiter oben angegebenen Anthelmintica weiter verarbeitet werden können.

Weitere Einzelheiten des Verfahrens der Erfindung sind den nachstehenden Beispielen zu entnehmen.

Beispiel 1

63 g Eisenpulver werden zur Aktivierung eine halbe Stunde mit 12 ml 5 n Salzsäure und 30 ml Wasser auf einem Wasserbad erwärmt. Danach setzt man 270 ml Wasser zu, stellt die Temperatur der Suspension auf 45 °C ein und fügt dann eine heisse Lösung von 58,5 g 4-Thiocyano-2-nitroanilin in 180 ml 96%igem Äthanol unter Rühren der erhaltenen Suspension zu. Die Temperatur des Reaktionsgemisches erhöht sich langsam. Sobald die Temperatur 75 °C erreicht, wird Eiskühlung eingesetzt, um die Temperatur im Reaktionsgemisch unter 85 °C zu halten. Die exotherme Phase der Reaktion dauert 3 bis 4 Minuten. Danach erhitzt man das Reaktionsgemisch so lange zum Sieden, bis die dünnschichtchromatographische Analyse ergibt, dass kein Ausgangsprodukt mehr vorhanden ist (etwa 15 Minuten). Dann wird das Reaktionsgemisch warm filtriert, das Filtrat mit 225 ml heissem Wasser versetzt, die erhaltene Lösung mit Aktivkohle entfärbt, warm filtriert und langsam, unter Rühren kristallisieren gelassen. Den Kristallbrei rührt man bei 0 °C weitere 5 bis 6 Stunden, saugt dann die Kristalle ab, wäscht sie mit kaltem 25%igem Äthanol und trocknet sie im Vakuum.

Es werden 41,6 g (79% Ausbeute) 4-Thiocyano-1,2-diamino-benzol erhalten. Fp.: 108 bis 110 °C.

Beispiel 2

Einer siedenden Suspension von 19,5 g 2-Nitro-4-thiocyano-anilin, 22,3 g Eisenpulver, 50 ml 96%igem Äthanol und 50 ml Wasser tropft man unter Rühren innerhalb von 5 Minuten eine Lösung von 2,2 g Ammoniumchlorid in 15 ml Wasser zu, rührt das erhaltene Reaktionsgemisch noch weitere 15 Minuten, filtriert es warm und gibt zu dem Filtrag 250 ml Wasser. Nach dem Abkühlen wird das ausgefallene kristalline 4-Thiocyano-1,2-diaminobenzol abfiltriert.

Ausbeute: 12,2 g (74%); Fp.: 108 bis 110 °C.

Beispiel 3

Einer siedenden Suspension von 19,5 g 2-Nitro-4-thiocyano-anilin, 22,3 g Eisenpulver, 40 ml 96%igem Äthanol und 50 ml Wasser tropft man unter Rühren innerhalb von 3 Minuten eine Lösung von 2,2 g Eisen(II)-chlorid in 4 ml Wasser zu, rührt das erhaltene Reaktionsgemisch noch weitere 10 Minuten, filtriert es und gibt zu dem Filtrat 200 ml Wasser. Nach dem Abkühlen wird das ausgefallene kristalline 4-Thiocyano-1,2-diaminobenzol abfiltriert. Ausbeute 12,3 g (74,5%); Fp.: 108 bis 110 °C.

Beispiel 4

16,5 g 1,2-Diamino-4-thiocyano-benzol löst man in 20 ml 5 n Salzsäure bei 80 °C, setzt die warme Lösung innerhalb von 10 Minuten einer 80 °C warmen Lösung von 13,7 g des Calciumsalzes des Carbomethoxy-cyanamids in 50 ml Wasser zu und erhöht die Temperatur des Reaktionsgemisches auf 93 bis 95 °C. Nach Beendigung der Zugabe wird der pH-Wert der Lösung kontrolliert und – wenn er vom pH-Wert 3 bis 4 differiert – mit 5 n Salzsäure auf diesen Wert eingestellt. Nach einigen Minuten beginnt die Kristallausscheidung, wobei durch zeitweise Zugabe von 5 n Salzsäure der pH-Wert zwischen 3 und 4 gehalten wird. Nach Beendigung der Säureaufnahme wird das Reaktionsgemisch noch 15 Minuten gerührt. Danach saugt man die Kristalle warm ab und wäscht sie zunächst mit Wasser und dann mit heissem Methanol.

Es werden 22,4 g (Ausbeute: 90,5%) 5(6)-Thiocyano-2-(methoxycarbonyl-amino)-benzimidazol erhalten. Molgewicht des Produktes: 248 (nach Bestimmung durch Massenspektrographie).

Beispiel 5

32 g technisches Calciumcyanamid (das 0,22 g Calciumcyanamid entspricht) werden in 114 ml Wasser eine Stunde bei 30 °C kräftig gerührt und danach bei dieser Temperatur innerhalb einer halben Stunde tropfenweise mit 20,5 ml (25,4 g; 0,269 Mol) Chlorameisensäuremethylester versetzt. Das erhaltene Reaktionsgemisch rührt man noch eine weitere Stunde, filtriert danach die Verunreinigungen ab, erwärmt das reine Filtrat auf 80 °C und fügt dann eine auf 80 °C erwärmte Lösung von 33 g (0,2 Mol) 1,2-Diamino-4-thiocyanobenzol in 40 ml 5 n Salzsäure zu. Das erhaltene Gemisch wird dann in der in Beispiel 6 angegebenen Weise weiter aufgearbeitet, wobei man 41 g (Ausbeute 83%) 5(6)-Thiocyano-2-methoxycarbonyl)-benzimidazol erhält. Das erhaltene Produkt ist identisch mit dem des Beispiels 6.

Beispiel 6

Es wird in der in Beispiel 7 beschriebenen Weise gearbeitet, jedoch mit dem Unterschied, dass das 1,2-Diamino-4-thiocyano-benzol in einem Gemisch von 50 ml 5 n Salzsäure und 40 ml 96%igem Äthanol gelöst der Carbomethoxy-cyanamid-Lösung zugesetzt wird. Nach Aufarbeiten des Gemisches in der in Beispiel 7 angegebenen Weise erhält man das 5(6)-Thiocyano-2-(methoxycarbonyl-amino)-benzimidazol in Form von gut ausgebildeten Kristallen.

Beispiel 7

8,25 g 2-Amino-4-thiocyano-anilin werden in 60 ml Aceton gelöst und danach unter Kühlen eine Lösung von 6,1 g Bromcyan in 20 ml Alkohol zugetropft. Das erhaltene Reaktionsgemisch lässt man über Nacht stehen, dampft es danach zur Trockene ein, nimmt den Rückstand in Wasser auf und stellt den pH-Wert der entstandenen Lösung mit 2 n Natriumhydroxydlösung auf 8 ein. Die ausgefallenen Kristalle werden abfiltriert, mit Wasser gewaschen und getrocknet.

Es werden 8,3 g 2-Amino-5-thiocyano-1H-benzimidazol erhalten; Fp.: 204 bis 205 °C (unter Zersetzung).

**Patentansprüche**

1. Verfahren zur Herstellung von 5(6)-Thiocyano-benzimidazol-Derivaten der allgemeinen Formel I

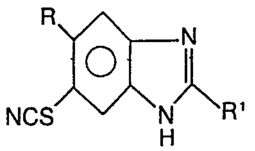

(I)

worin

R Wasserstoff, Halogen, $C_{1-4}$-Alkyl, $C_{1-3}$-Alkoxy oder Trifluormethyl,

$R^1$ Amino oder $C_{1-4}$-Alkoxycarbonylamino bedeuten, sowie deren Salzen,

dadurch gekennzeichnet, dass man eine Verbindung der allgemeinen Formel II

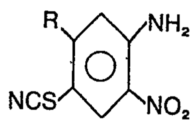

(II)

worin

R die oben angegebene Bedeutung hat,

mit Eisen zur Verbindung der allgemeinen Formel III

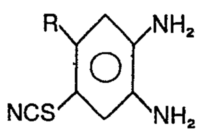

(III)

worin

R die oben angegebene Bedeutung hat,

selektiv reduziert, die entweder mit einem Cyanamid der allgemeinen Formel IV

$$R^3 - NHCN \qquad (IV)$$

worin

$R^3$ Wasserstoff oder $C_{1-4}$-Alkoxycarbonyl bedeutet,

oder mit einem Halogencyan der allgemeinen Formel V

$$HlgCN \qquad (V)$$

worin

Hlg Chlor oder Brom bedeutet,

umgesetzt wird.

2. Verfahren nach Anspruch 1, dadurch gekennzeichnet, dass man die Reduktion der Verbindungen der allgemeinen Formel II mit Eisen in einem wässrigen-organischen, neutralen oder schwach sauren Medium durchführt.

3. Verfahren nach Anspruch 1 oder 2, dadurch gekennzeichnet, dass man für die Reduktion in feinteiliger Form vorliegendes Eisen verwendet, das zuvor einer Aktivierungsbehandlung mit einer der starken anorganischen Säuren oder deren Salzen unterworfen worden ist.

4. Verfahren nach Anspruch 1, 2 oder 3, dadurch gekennzeichnet, dass man die Reduktion bei Temperaturen zwischen 20 und 100 °C durchführt.

5. Verfahren nach Anspruch 4, dadurch gekennzeichnet, dass man die Reduktion bei Temperaturen zwischen 70 und 100 °C durchführt.

6. Verfahren nach Anspruch 1 bis 5, dadurch gekennzeichnet, dass man die Umsetzung der Verbindung der allgemeinen Formel III mit einem Cyanamid der allgemeinen Formel IV in einem wässrigen oder organischen Lösungsmittelmedium in Gegenwart einer Säure durchführt.

**Claims**

1. Process for preparing 5(6)-thiocyano-benzimidazole derivatives of general formula I

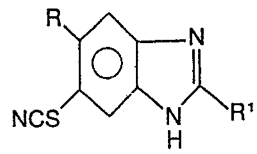

(I)

wherein

R represents hydrogen, halogen, $C_{1-4}$ alkyl, $C_{1-3}$ alkoxy or trifluoromethyl,

$R^1$ represents amino or $C_{1-4}$ alkoxycarbonylamino, and the salts thereof,

characterised in that a compound of general formula II

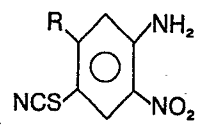

(II)

wherein

R is as hereinbefore defined,

is selectively reduced with iron to yield the compound of general formula III

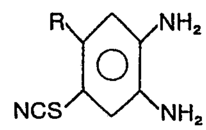

(III)

wherein

R is as hereinbefore defined,

which is reacted either with a cyanamide of general formula IV

$$R^3 - NHCN \qquad (IV)$$

wherein

$R^3$ represents hydrogen or $C_{1-4}$ alkoxycarbonyl, or with a halocyanogen of general formula V

$$HlgCN \qquad (V)$$

wherein Hlg represents chlorine or bromine.

2. Process as claimed in claim 1, characterised in that the reduction of the compounds of general formula II with iron is carried out in an aqueous-organic, neutral or weakly acidic medium.

3. Process as claimed in claim 1 or 2, characterised in that the reduction is carried out using finely divided iron which has previously been subjected to an activation treatment with one of the strong inorganic acids or the salts thereof.

4. Process as claimed in claim 1, 2 or 3, characterised in that the reduction is carried out at temperatures of between 20 and 100 °C.

5. Process as claimed in claim 4, characterised in that the reduction is carried out at temperatures of between 70 and 100 °C.

6. Process as claimed in claims 1 to 5, characterised in that the reaction of the compound of general formula III with a cyanamide of general formula IV is carried out in an aqueous or organic solvent medium in the presence of an acid.

**Revendications**

1. Procédé de préparation de dérivés du 5(6)-thiocyano-benzimidazole de formule générale I

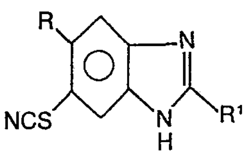

(I)

dans laquelle

R représente l'hydrogène, un halogène, un groupe alkyle en C1–C4, alcoxy en C1–C3 ou trifluorométhyle,

$R^1$ représente un groupe amino ou (alcoxy en C1–C4)-carbonylamino, et de leurs sels,
caractérisé en ce que l'on soumet un composé de formule générale II

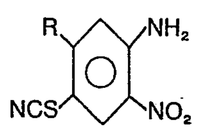

(II)

dans laquelle R a la signification indiquée ci-dessus, à réduction sélective à l'aide du fer, ce qui donne un composé de formule générale III

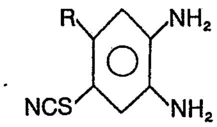

(III)

dans laquelle R a la signification indiquée ci-dessus, qu'on fait réagir soit avec un cyanamide de formule générale IV

$$R^3 - NHCN \qquad (IV)$$

dans laquelle $R^3$ représente l'hydrogène ou un groupe (alcoxy en C1–C4)-carbonyle,

soit avec un halogénocyanogène de formule générale V

$$HlgCN \qquad (V)$$

dans laquelle Hlg représente le chlore ou le brome.

2. Procédé selon la revendication 1, caractérisé en ce que la réduction des composés de formule générale II à l'aide du fer est effectuée dans un milieu aqueux-organique, neutre ou légèrement acide.

3. Procédé selon la revendication 1 ou 2, caractérisé en ce que l'on utilise pour la réduction du fer à l'état de fine division soumis au préalable à un traitement d'activation par un acide minéral fort ou un sel d'un tel acide.

4. Procédé selon la revendication 1, 2 ou 3, caractérisé en ce que la réduction est effectuée à des températures de 20 à 100 °C.

5. Procédé selon la revendication 4, caractérisé en ce que la réduction est effectuée à des températures de 70 à 100 °C.

6. Procédé selon les revendications 1 à 5, caractérisé en ce que la réaction du composé de formule générale III avec un cyanamide de formule générale IV est effectuée dans un milieu solvant aqueux ou organique en présence d'un acide.